# EUROPEAN PATENT APPLICATION

(11) **EP 3 489 225 A1**
(43) Date of publication of application: **29.05.2019**
(21) Application number: 17830514.0
(22) Date of filing: 24.07.2017
(51) Int. Cl.: C07D 295/096, C07D 295/205, C07D 295/185, C07D 295/192, C07C 321/26, C07C 319/02, A61K 31/495, A61P 25/24

(54) **VORTIOXETINE ANALOGUE AND USE AND PREPARATION THEREOF**

(30) Priority: 22.07.2016 CN 201610584432
(71) Applicant: Jiangsu Nhwaluokang Pharmaceutical Research and Development Co., Ltd., Xuzhou, Jiangsu 221000 (CN)
(72) Inventor: WANG, Tao, Chongqing 401332 (CN); LIAO, Jian, Chongqing 401332 (CN); XU, Xiangqing, Chongqing 401332 (CN); REN, Liang, Chongqing 401332 (CN); LI, Qingeng, Chongqing 401332 (CN)
(74) Representative: Michalski Hüttermann & Partner Patentanwälte mbB
(86) International application number: PCT/CN2017/094037
(87) International publication number: WO 2018/014880

(57) **Abstract**

The present invention relates to a new type of vortioxetine analogue of Formula I or its polymorph or solvate, a compositions and kit comprising the analogue, the polymorph or the solvate, and use of the compound or the polymorph or the solvate in the preparation of a medicament for the treatment of depression, and a preparation method of the analogue, and intermediate compounds in the method.

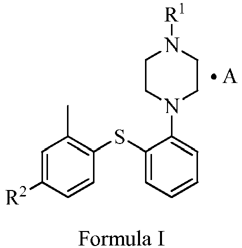

## Description

### CROSS REFERENCE TO RELATED APPLICATIONS

This application claims the priority of Chinese Patent Application No. CN 201610584432.0, filed on Jul. 22, 2016. The disclosure of the application is hereby incorporated herein by reference in its entirety.

### Technical Field

The invention belongs to the field of chemical pharmacy. In particular, the invention relates to a novel vortioxetine analogue or a polymorph or a solvate thereof, a composition and a kit comprising the analogue, polymorph or solvate, and the use of the analogue, polymorph or solvate in the preparation of a drug for treatment of depression. The invention also relates to a method for preparing the analogue, and an intermediate in the method.

### Background Art

Vortioxetine, developed by H. Lundbeck A/S [Denmark] and Takeda Pharmaceutical Company Limited [Japan], is indicated for the treatment of patients suffering from Major Depression Disorder (MDD) and has been approved for marketing in the United States since September of 2013. The structure of vortioxetine hydrobromide is as follows:

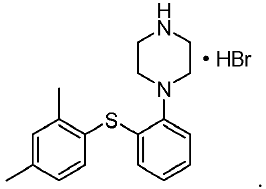

Vortioxetine is well absorbed by oral administration with a bioavailability of about 75%. It was shown by experiments that the distribution concentration in brain is only 2.77 µg/g, lower than those in liver (22.3 µg/g), lung (15.5 µg/g) and kidney (10.3 µg/g), 2h after male Listar Hooded rats were orally administered with ¹⁴C-vortioxetine (20 mg/kg) (see FDA PHARMACOLOGY REVIEW(S), pp. 24). It was demonstrated from the above data that vortioxetine was able to penetrate the blood brain barrier (BBB), but weak penetration ability was one of its shortcomings.

In addition, it was also found by experiments that metabolites were dominant and parent drug accounted for about 8% in plasma in 4 hours after ¹⁴C-vortioxetine free base (50 mg) was orally administered by human (see FDA PHARMACOLOGY REVIEW(S), pp. 26). In these metabolites, it was worth noting that the product LUAA34443 from oxidation of the 4-position methyl group on the aromatic ring of vortioxetine and the LUAA34443 glucuronide accounted for 44%. The *vivo* metabolic process of vortioxetine free base is shown as below:

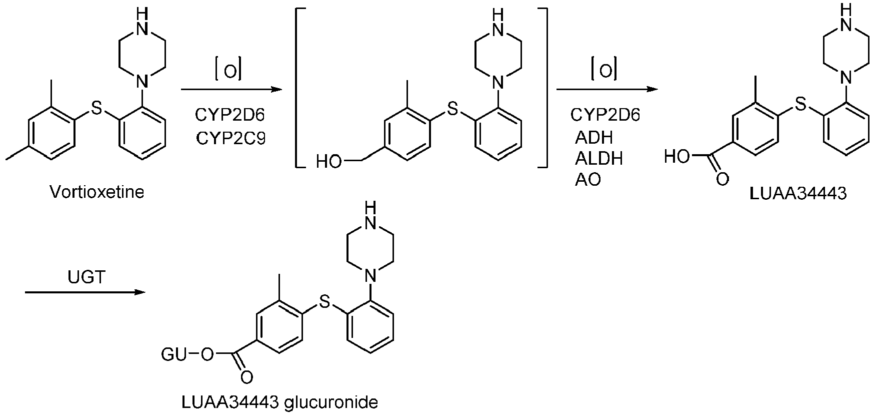

It is obvious that the antidepressant effect fails to function as the LUAA34443 and the LUAA34443 glucuronide are not able to penetrate the blood brain barrier. Therefore, it is necessary to modify the structure of vortioxetine to improve its ability of penetrating the blood brain barrier and its metabolic stability.

### Summary of the Invention

According to the first aspect of the invention, it provides a novel vortioxetine analogue or a polymorph or a solvate thereof. Compared with vortioxetine, the vortioxetine analogue of the invention has the advantages not only of better antidepressant activity but also of improved lipid-water partition coefficient, higher ability of penetrating the blood brain barrier, and higher metabolic stability. This means that the vortioxetine analogue of the invention may be administered with a less amount of dosage and a longer duration of action.

The vortioxetine analogue of the invention has a structure of the following formula I: 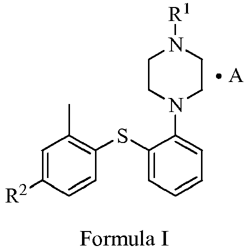 wherein:
R¹ represents H;
R² represents halogen or C₁₋₆ alkyl group substituted by one or more halogens; and
A is absent or a pharmaceutically acceptable inorganic acid or organic acid.

According to the second aspect of the invention, it provides a pharmaceutical composition, comprising the vortioxetine analogue of the invention or polymorph or solvate thereof and one or more pharmaceutically acceptable carrier(s).

According to the third aspect of the invention, it provides a kit, comprising the vortioxetine analogue of the invention or polymorph or solvate thereof or the pharmaceutical composition of the invention.

According to the fourth aspect of the invention, it provides a method for treatment of depression disorder (particularly Major Depression Disorder), including administering an effective amount of the vortioxetine analogue of the invention or polymorph or solvate thereof to an individual in need thereof.

According to the fifth aspect of the invention, it provides the vortioxetine analogue of the invention or polymorph or solvate thereof for treatment of depression disorder, particularly Major Depression Disorder.

According to the sixth aspect of the invention, it provides a use of the vortioxetine analogue of the invention or polymorph or solvate thereof in the preparation of a medicament for treatment of depression (particularly Major Depression Disorder).

According to the seventh aspect of the invention, it provides a method for preparing the vortioxetine analogue of the invention, including the following steps: 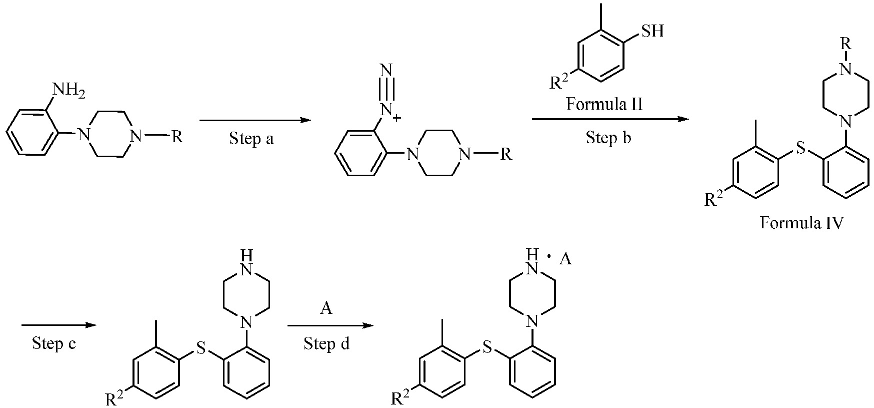 or, 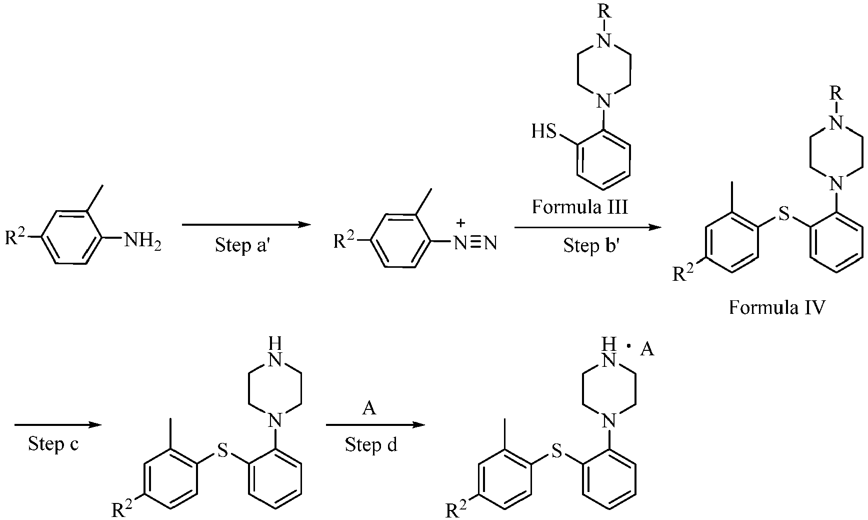 wherein,
R represents an amino protective group;
A and R² are as defined above; and
when A is absent, the reaction in step d is not carried out.

According to the eighth aspect of the invention, it provides an intermediate for preparing the vortioxetine analogue of the invention, the intermediate having a structure of the following formula IV: 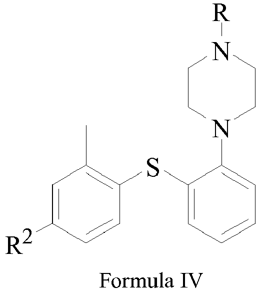 wherein:
R represents an amino protective group; and
R² represents halogen or C₁₋₆ alkyl group substituted by one or more halogens.

According to the ninth aspect of the invention, it provides a raw material for preparing the vortioxetine analogue of the invention, the raw material having a structure of the following formula II or formula III: 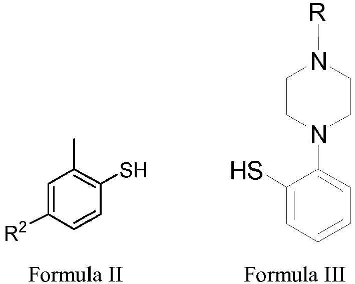 wherein,
R represents an amino protective group; and
R² represents halogen or C₁₋₆ alkyl group substituted by one or more halogens.

According to the tenth aspect of the invention, it provides a method for preparing a compound of formula II, including the following steps: 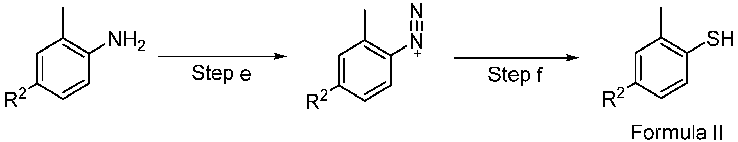 wherein R² is as defined above.

According to the eleventh aspect of the invention, it provides a method for preparing a compound of formula III, including the following steps: 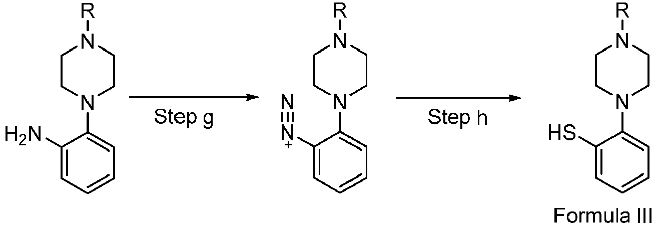 wherein R is as defined above.

### Detailed Description of the Invention

### Definitions

All technical and scientific terms used herein are intended to have the same meaning as commonly understood by those skilled in the art, unless otherwise defined hereinafter. Reference to the technology used herein is intended to refer to the technology commonly understood in the art, including those variations of technology or alternatives of equivalent technology that are readily apparent to those skilled in the art. While it is believed that the following terms are well understood by those skilled in the art, the following definitions are set forth to better construe the invention.

As used herein, the terms "including," "comprising," "having," "containing" or "ralating to" and other variations thereof herein are inclusive or open-ended and do not exclude additional elements or method steps not listed.

As used herein, the term "substituted" means that one or more (e.g., 1, 2, 3 or 4) H of the designated atom is selectively replaced by the specified group, provided that the normal valence of the designated atom in the existing circumstances is not exceeded and that the substitution results in a stable compound. The combination of a substituent and/or a variable is allowed only when such a combination results in a stable compound.

As used herein, the term "protective group" means that when a multifunctional organic compound is subjected to a reaction, the other groups are protected before the reaction and recovered after the reaction is completed, in order to make the reaction occur only at the desired group and to prevent the other groups from being affected. An agent being able to protect a kind of group is called a protective group for the group. Selection of an appropriate protective group can be readily determined by those skilled in the art. Protecting group chemistry can be found, e.g. in T.W. Greene and P.G.M. Wuts, Protective Groups in Organic Synthesis, 3rd Edition, Wiley & Sons, Inc., New York (1999), which is hereby incorporated herein by reference in its entirety.

As used herein, the term "amino protective group" refers to a protective group, which prevents the amino from occurring the undesired chemical rection. The amino protective groups include but not limited to tert-butoxycarbonyl, benzyloxycarbonyl, 9-fluorenylmethoxycarbonyl, allyloxycarbonyl, trimethylsilylethoxycarbonyl, methoxycarbonyl, ethoxycarbonyl, phthaloyl, *p*-toluenesulfonyl, *o*-nitrophenylsulfonyl, *p*-nitrophenylsulfonyl, formyl, acetyl, trifluoroacetyl, propionyl, pivaloyl, benzoyl, triphenylmethyl, benzyl, 2,4-dimethoxybenzyl, *p*-methoxybenzyl and the like. Selection of an appropriate amino protective group can be readily determined by those skilled in the art.

As used herein, the term "C₁₋₆ alkyl group" refers to a saturated straight chain or branched chain hydrocarbyl group having 1-6 carbon atoms, such as methyl, ethyl, propyl, isopropyl, n-butyl, isobutyl, sec-butyl, tert-butyl, n-pentyl, isopentyl, neopentyl, n-hexyl, isohexyl, etc.

As used herein, the term "halogenated" or "halogen" group is defined to include F, Cl, Br, or I.

As used herein, the term "pharmaceutically acceptable acid" refers to an acid that is compatible with pharmaceutical practice and animal use from the perspective of biology, preparation and formulation. The pharmaceutically acceptable acids include an inorganic acid, such as hydrochloric acid, hydrobromic acid, hydroiodic acid, sulfuric acid, disulfuric acid, nitric acid, boric acid, phosphoric acid, carbonic acid and any combination thereof; and an organic acid, such as formic acid, acetic acid, propionic acid, acetoacetic acid, trifluoroacetic acid, propionic acid, pyruvic acid, butyric acid, hexanoic acid, heptanoic acid, hendecanoic acid, lauric acid, stearic acid, palmitic acid, oxalic acid, malonic acid, succinic acid, glutaric acid, adipic acid, maleic acid, fumaric acid, lactic acid, malic acid, citric acid, tartaric acid, benzoic acid, salicylic acid, cinnamic acid, naphthoic acid, pamoic acid, nicotinic acid, orotic acid, methyl sulfuric acid, dodecyl sulfuric acid, methanesulfonic acid, trifluoromethanesulfonic acid, ethanesulfonic acid, ethanedisulfonic acid, hydroxyethanesulphonic acid, benzenesulfonic acid, *p*-toluenesulfonic acid, 1,5-naphthalenedisulfonic acid, naphthalene-2-sulfonic acid, camphorsulfonic acid, sulfamic acid, glutamic acid, aspartic acid, gluconic acid, glucuronic acid and any combination thereof.

The vortioxetine analogue of the invention can exist in the forms as a crystalline or a polymorph, which can be a single polymorph or a mixture of more than one polymorph in any ratio.

The vortioxetine analogue of the invention can exist in the form of a solvate thereof, in particular a hydrate, wherein the vortioxetine analogue of the invention comprises a polar solvent, such as water, ethanol, isopropanol, ethyl acetate or acetone, as a structural element of the crystal lattice of the analogue. The amount of the polar solvent, particularly water, can be present in a stoichiometric ratio or a non-stoichiometric ratio.

### Compound and preparation method

An object of the present invention is to provide a series of novel vortioxetine analogues. The major structure difference between the vortioxetine analogue and vortioxetine is that in vortioxetine the 4-position of the benzene ring on the left side is methyl group, while in the vortioxetine analogue of the invention the corresponding position is halogen or alkyl group substituted by one or more halogens. The vortioxetine analogue of the invention has an improved molecular lipid-water partition coefficient and a higher ability of penetrating the blood brain barrier with maintained antidepressant activity, and can penetrate the blood brain barrier more rapidly after oral administration to take effect more quickly. Meanwhile, compared with methyl group, halogen or alkyl group substituted by one or more halogens is less likely to undergo oxidative metabolism *in vivo,* so the vortioxetine analogue of the invention has a higher metabolic stability and a longer duration of action in comparison to vortexine, and accordingly the dosage and the frenquency of administration can be decreased. Therefore, the vortioxetine analogue of the invention overcomes the shortcomings of the weak ability of penetrating the blood brain barrier and the poor metabolic stability for vortioxetine.

In particular, the invention provides a compound of formula I or a polymorph or a solvate thereof. 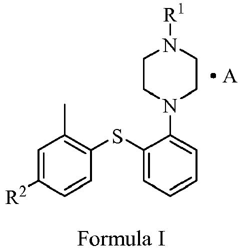 wherein:
R¹ represents H;
R² represents halogen or C₁₋₆ alkyl group substituted by one or more halogens; and
A is absent or a pharmaceutically acceptable inorganic acid or organic acid.

According to an embodiment of the invention, R² represents C₁₋₆ alkyl group substituted by one or more halogens. In a preferred embodiment, R² represents C₁₋₆ alkyl group substituted by one or more fluorines, such as CF₃, C₂F₅, CHF₂, CH₂F, CF₂CH₃, or CF₂CH₂CH₃, etc. In a particularly preferred embodiment, R² represents CF₃ (trifluoromethyl).

According to an embodiment of the invention, A is absent.

According to another embodiment of the invention, A is a pharmaceutically acceptable inorganic acid or organic acid. In a preferred embodiment, A is an inorganic acid selected from hydrochloric acid, hydrobromic acid, hydroiodic acid, sulfuric acid, disulfuric acid, nitric acid, boric acid, phosphoric acid and carbonic acid, or an organic acid selected from formic acid, acetic acid, propionic acid, acetoacetic acid, trifluoroacetic acid, propionic acid, pyruvic acid, butyric acid, hexanoic acid, heptanoic acid, hendecanoic acid, lauric acid, stearic acid, palmitic acid, oxalic acid, malonic acid, succinic acid, glutaric acid, adipic acid, maleic acid, fumaric acid, lactic acid, malic acid, citric acid, tartaric acid, benzoic acid, salicylic acid, cinnamic acid, naphthoic acid, pamoic acid, nicotinic acid, orotic acid, methyl sulfuric acid, dodecyl sulfuric acid, methanesulfonic acid, trifluoromethanesulfonic acid, ethanesulfonic acid, ethanedisulfonic acid, hydroxyethanesulphonic acid, benzenesulfonic acid, *p*-toluenesulfonic acid, 1,5-naphthalenedisulfonic acid, naphthalene-2-sulfonic acid, camphorsulfonic acid, sulfamic acid, glutamic acid, aspartic acid, gluconic acid and glucuronic acid. In a particularly preferred embodiment, A is an inorganic acid selected from hydrochloric acid, hydrobromic acid and sulfuric acid, or an organic acid selected from citric acid, methanesulfonic acid, ethanesulfonic acid, benzenesulfonic acid and *p*-toluenesulfonic acid.

The invention encompasses compounds of formula I obtained by any combination of the above preferred groups.

According to an embodiment of the invention, the compound of formula I is a compound of the following formula I-1: 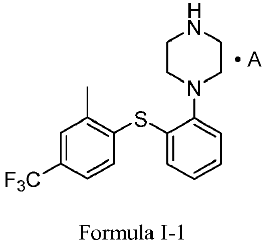 wherein A is as defined above.

According to an embodiment of the invention, the vortioxetine analogue of the invention is selected from:

| No. | Name | Structure | A | R¹ |
|---|---|---|---|---|
| 1 | 1-[2-(2-methyl-4-trifluoromethyl phenylthio)phenyl]piperazine | 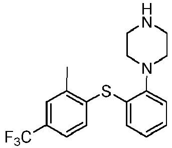 | absent | H |
| 2 | 1-[2-(2-methyl-4-trifluoromethyl phenylthio)phenyl]piperazine hydrochloride | 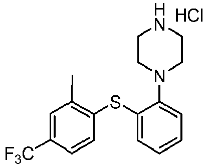 | HCl | H |
| 3 | 1-[2-(2-methyl-4-trifluoromethyl phenylthio)phenyl]piperazine hydro bromide | 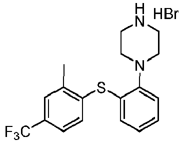 | HBr | H |
| 4 | 1-[2-(2-methyl-4-trifluoromethyl phenylthio)phenyl]piperazine sulfate | 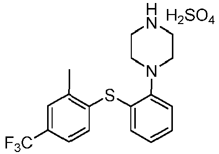 | H₂SO₄ | H |
| 5 | 1-[2-(2-methyl-4-trifluoromethyl phenylthio)phenyl]piperazine methanesulfonate | 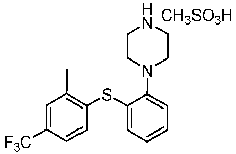 | methane sulfonic acid | H |
| 6 | 1-[2-(2-methyl-4-trifluoromethyl phenylthio)phenyl]piperazine ethanesulfonate | 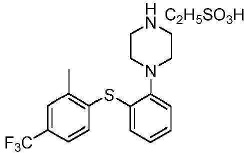 | ethane sulfonic acid | H |
| 7 | 1-[2-(2-methyl-4-trifluoromethyl phenylthio)phenyl]piperazine benzenesulfonate | 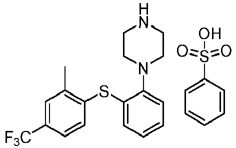 | benzene sulfonic acid | H |
| 8 | 1-[2-(2-methyl-4-trifluoromethyl phenylthio)phenyl]piperazine p-toluenesulfonate | 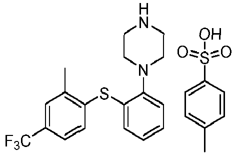 | p-toluene sulfonic acid | H |
| 9 | 1-[2-(2-methyl-4-trifluoromethyl phenylthio)phenyl]piperazine citrate | 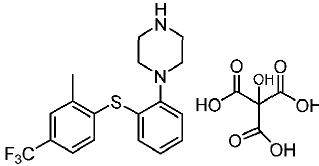 | citrate | H |

Another object of the present invention is to provide a method for preparing the vortioxetine analogue of the invention, including the following steps: 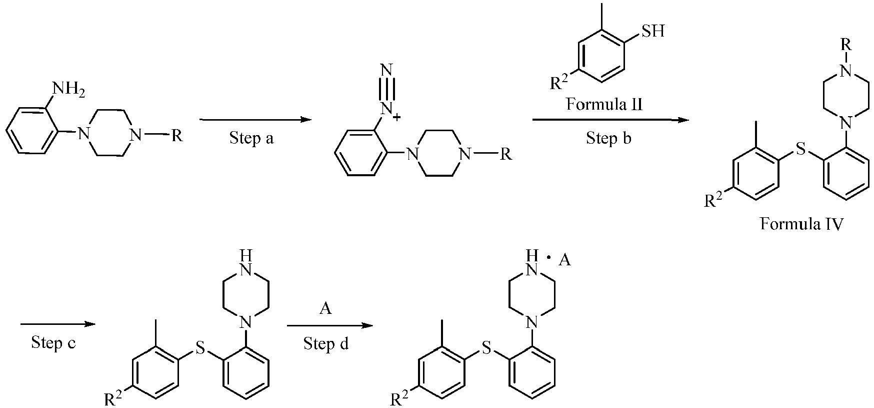 or, 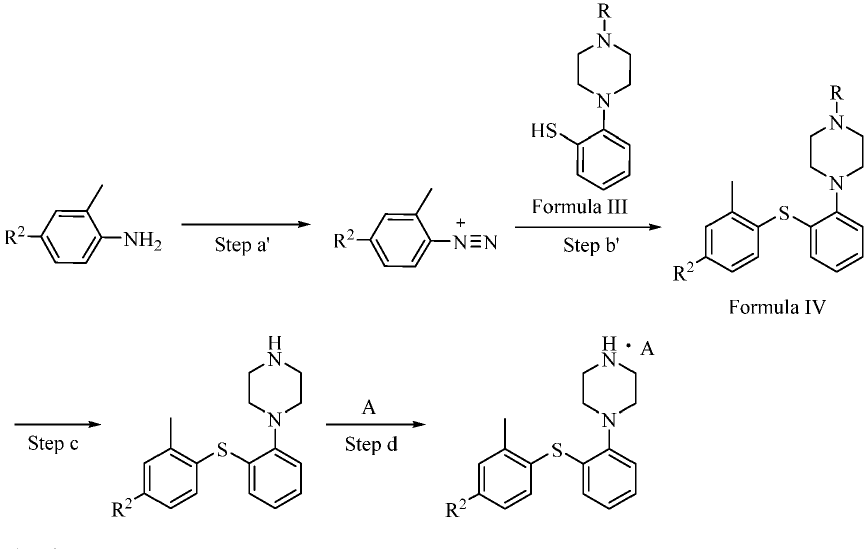 wherein,
R represents an amino protective group;
A and R² are as defined above; and
when A is absent, the reaction in step d is not carried out.

According to an embodiment of the invention, R represents an amino protective group selected from tert-butoxycarbonyl, benzyloxycarbonyl, 9-fluorenylmethoxycarbonyl, allyloxycarbonyl, trimethylsilylethoxycarbonyl, methoxycarbonyl, ethoxycarbonyl, phthaloyl, *p*-toluenesulfonyl, *o*-nitrophenylsulfonyl, *p*-nitrophenylsulfonyl, formyl, acetyl, trifluoroacetyl, propionyl, pivaloyl, benzoyl, triphenylmethyl, benzyl, 2,4-dimethoxybenzyl and *p*-methoxybenzyl. In one preferred embodiment, R represents an amino protective group selected from tert-butoxycarbonyl, benzyloxycarbonyl, 9-fluorenylmethoxycarbonyl, acetyl, propionyl and benzoyl.

### Step a and step a':

According to an embodiment of the invention, the diazotization reaction in step a and step a' is carried out in the presence of an acid (e.g., hydrochloric acid, sulfuric acid or nitric acid) and a nitrite (e.g., sodium nitrite or potassium nitrite).

In a preferred embodiment, the diazotization reaction is carried out in a mixed solvent of an alcohol (e.g., methanol or ethanol) and water at a relatively low temperature (e.g., -10°C to 10°C, preferably 0-5°C).

### Step b and step b':

According to one embodiment of the invention, the reaction in step b and step b' is carried out in a mixed solvent of an alcohol (e.g., methanol or ethanol) and water at a relatively high temperature (e.g., 30-100°C, preferably 50-60°C).

### Step c:

According to an embodiment of the invention, the reaction in step c is carried out through a conventional method of removing the amino protective group, and the specific method used depends on the selected amino protective group. For example, tert-butoxycarbonyl can be removed by an acid (e.g., HCl, trifluoroacetic acid, etc) treatment; benzyloxycarbonyl can be removed by catalytic hydrogenation on palladium (e.g., Pd-C, etc); 9-fluorenylmethoxycarbonyl can be removed by an organic base (e.g., triethylamine, piperidine, etc) treatment; and the like.

### Step d:

According to an embodiment of the invention, the reaction in step d is carried out in an alcohol (e.g., methanol, ethanol, propanol, etc) solution at a temperature in the range of 0°C (with ice bath) to the reflux temperature of the solvent.

Another object of the present invention is to provide an intermediate for preparing the vortioxetine analogue of the invention, the intermediate having a structure of the following formula IV: 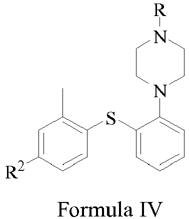 wherein:
R represents an amino protective group; and
R² represents halogen or C₁₋₆ alkyl group substituted by one or more halogens.

According to an embodiment of the invention, R is an amino protecting group selected from tert-butoxycarbonyl, benzyloxycarbonyl, 9-fluorenylmethoxycarbonyl, allyloxycarbonyl, trimethylsilyloxycarbonyl, methoxycarbonyl, ethoxycarbonyl, phthaloyl, *p*-toluenesulfonyl, *o*-nitrophenylsulfonyl, *p*-nitrophenylsulfonyl, formyl, acetyl, trifluoroacetyl, propionyl, pivaloyl, benzoyl, triphenylmethyl, benzyl, 2,4-dimethoxybenzyl and *p*-methoxybenzyl. In a preferred embodiment, R is an amino protecting group selected from tert-butoxycarbonyl, benzyloxycarbonyl, 9-fluorenylmethoxycarbonyl, acetyl, propionyl and benzoyl.

According to an embodiment of the invention, R² is C₁₋₆ alkyl substituted by one or more halogens. In a preferred embodiment, R² is C₁₋₆ alkyl substituted by one or more fluorines, such as CF₃, C₂F₅, CHF₂, CH₂F, CF₂CH₃, or CF₂CH₂CH₃, etc. In a particularly preferred embodiment, R² is CF₃ (trifluoromethyl).

The invention encompasses the compound of formula IV obtained by any combination of the above preferred groups.

According to an embodiment of the invention, the compound of formula IV is a compound of the following formula IV-1:

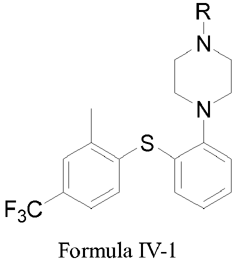

According to one embodiment of the invention, the compound of formula IV is selected from the group consisting of:

| No. | Name | Structure | R |
|---|---|---|---|
| 16 | 1 -tert-Butoxycarbonyl-4-[2-(2-methyl-4-trifluoromethylphenylthio) phenyl]piperazine | 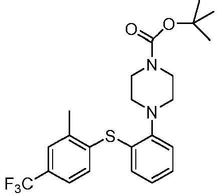 | tert-butoxy carbonyl |
| 17 | 1 -benzyloxycarbonyl-4-[2-(2-methyl-4-trifluoromethylphenylthio) phenyl]piperazine | 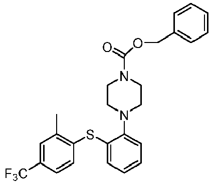 | benzyloxy carbonyl |
| 18 | 1-(9-fluorenylmethoxycarbonyl)-4-[2-(2-methyl-4-trifluoromethyl phenylthio)phenyl]piperazine | 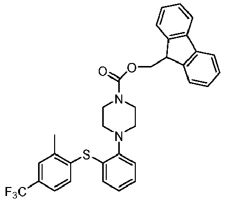 | 9-fluorenyl methoxy carbonyl |
| 19 | 1 -acetyl-4-[2-(2-methyl-4-trifluoro methylphenylthio)phenyl]piperazine | 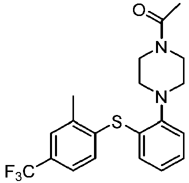 | acetyl |
| 20 | 1-propionyl-4-[2-(2-methyl-4-trifluoromethylphenylthio)phenyl] piperazine | 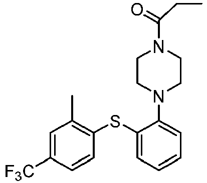 | propionyl |
| 21 | 1-benzoyl-4-[2-(2-methyl-4-trifluoromethylphenylthio)phenyl] piperazine | 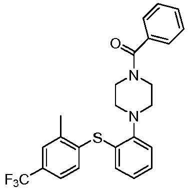 | benzoyl |

Another object of the invention is to provide a raw material for preparing the vortioxetine analogue of the invention, which has the structure of the following formula II, 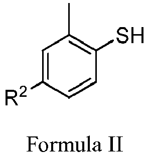 wherein R² is halogen or C₁₋₆ alkyl substituted by one or more halogens.

According to an embodiment of the invention, R² is C₁₋₆ alkyl substituted by one or more halogens. In a preferred embodiment, R² is C₁₋₆ alkyl substituted by one or more fluorines, such as CF₃, C₂F₅, CHF₂, CH₂F, CF₂CH₃, or CF₂CH₂CH₃, etc. In a particularly preferred embodiment, R² is CF₃ (trifluoromethyl).

According to an embodiment of the invention, the compound of formula II is a compound of the following formula II-1:

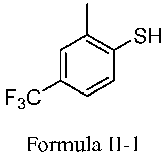

According to an embodiment of the invention, the compound of formula II is prepared by the following method: 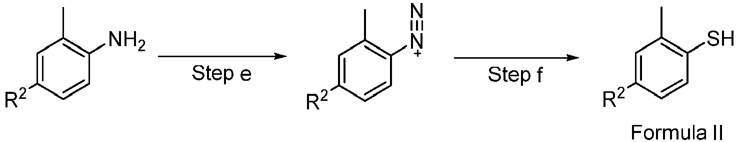 wherein R² is as defined above.

### Step e:

According to an embodiment of the invention, the diazotization reaction in step e is carried out in the presence of an acid such as hydrochloric acid, sulfuric acid or nitric acid and a nitrite such as sodium nitrite or potassium nitrite.

In a preferred embodiment, the diazotization reaction is carried out in a mixed solvent of an alcohol such as methanol or ethanol and water at a relatively low temperature, for example, -10 °C to 10 °C, preferably 0 to 5 °C.

### Step f:

According to an embodiment of the invention, the reaction in step f is performed in the presence of an alkyl xanthate salt (wherein the alkyl is preferably C₁₋₆ alkyl), the salt is preferably an alkali metal salt (such as lithium salt, sodium salt or a potassium salt, etc). An example of the reaction is carried out in the presence of potassium ethyl xanthate as the alkyl xanthate and a strong base such as sodium hydroxide or potassium hydroxide.

In a preferred embodiment, the alkyl xanthate salt and the strong base are added to the reaction simultaneously or sequentially.

Another object of the invention is to provide a raw material for preparing the vortioxetine analogue of the invention, which has the structure of the following formula III, 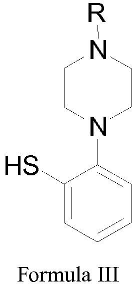 wherein R is amino protecting group.

According to an embodiment of the invention, R is an amino protecting group selected from tert-butoxycarbonyl, benzyloxycarbonyl, 9-fluorenylmethoxycarbonyl, allyloxycarbonyl, trimethylsilylethoxycarbonyl, methoxycarbonyl, ethoxycarbonyl, phthaloyl, *p*-toluenesulfonyl, *o*-nitrophenylsulfonyl, *p*-nitrophenylsulfonyl, formyl, acetyl, trifluoroacetyl, propionyl, pivaloyl, benzoyl, triphenylmethyl, benzyl, 2,4-dimethoxybenzyl and *p*-methoxybenzyl. In a preferred embodiment, R is an amino protecting group selected from tert-butoxycarbonyl, benzyloxycarbonyl, 9-fluorenylmethoxycarbonyl, acetyl, propionyl and benzoyl.

According to an embodiment of the invention, the compound of formula III is selected from:

| No. | Name | Structure | R |
|---|---|---|---|
| 10 | 1-tert-butoxycarbonyl-4-[2-(mercapto) phenyl]piperazine | 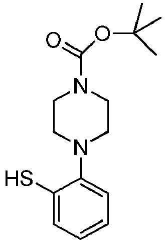 | tert-butoxy carbonyl |
| 11 | -benzyloxycarbonyl-4-[2-(mercapto)phenyl] piperazine | 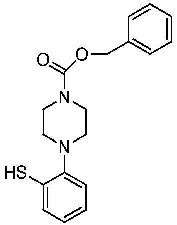 | benzyloxy carbonyl |
| 12 | 1-(9-fluorenylmethoxycarbonyl)-4-[2-(mercapto)phenyl]piperazine | 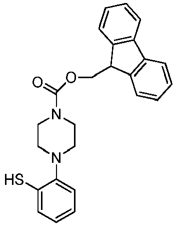 | 9-fluorenyl methoxycarbonyl |
| 13 | 1-acetyl-4-[2-(mercapto)phenyl]piperazine | 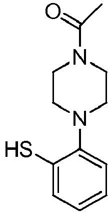 | acetyl |
| 14 | 1-propionyl-4-[2-(mercapto)phenyl] piperazine | 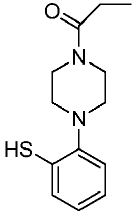 | propionyl |
| 15 | 1-benzoyl-4-[2-(mercapto)phenyl]piperazine | 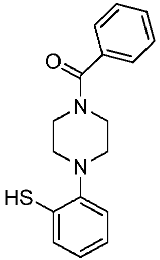 | benzoyl |

According to an embodiment of the invention, the compound of formula III is prepared by the following method: 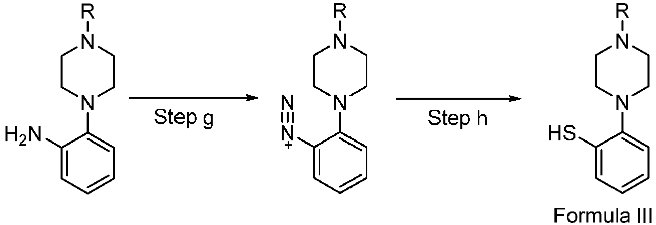 wherein R is as defined above;

### Step g:

According to an embodiment of the invention, the diazotization reaction in step g is carried out in the presence of an acid such as hydrochloric acid, sulfuric acid or nitric acid and a nitrite such as sodium nitrite or potassium nitrite.

In a preferred embodiment, the diazotization reaction is carried out in a mixed solvent of an alcohol such as methanol or ethanol and water at a relatively low temperature, for example, -10 °C to 10 °C, preferably 0 to 5 °C.

### Step h:

According to an embodiment of the invention, the reaction in step f is in the presence of an alkyl xanthate salt (wherein the alkyl is preferably C₁₋₆ alkyl), the salt is preferably an alkali metal salt (such as lithium salt, sodium salt or a potassium salt, etc). An example of the reaction is carried out in the presence of potassium ethyl xanthate as the alkyl xanthate salt and a strong base such as sodium hydroxide or potassium hydroxide.

In a preferred embodiment, the alkyl xanthate salt and the strong base are added to the reaction simultaneously or sequentially.

### Pharmaceutical composition and kit

Another object of the present invention is to provide a pharmaceutical composition comprising the vortioxetine analogue of the invention or polymorph or solvate thereof and one or more pharmaceutically acceptable carriers.

As used herein, the term "a pharmaceutically acceptable carrier" means a diluent, adjuvant, excipient or vehicle with which the therapeutic agent is administered and which is suitable for contacting the tissues of humans and/or other animals without excessive toxicity, irritation, allergic reactions or other problems or complications corresponding to a reasonable benefit/risk ratio in the scope of reasonable medical judgment.

The pharmaceutically acceptable carrier that can be used in the pharmaceutical compositions of the invention includes, but not limited to, sterile liquids such as water and oils, including those of petroleum, animal, vegetable or synthetic origin, such as peanut oil, soybean oil, mineral oil, sesame oil, etc. Water is an exemplary carrier when the pharmaceutical composition is administered intravenously. Physiological saline, glucose and glycerin aqueous solution are as a liquid carrier, particularly for injection. Suitable pharmaceutical excipient includes starch, glucose, lactose, sucrose, gelatin, maltose, chalk, silica gel, sodium stearate, glycerylmonostearate, talc, sodium chloride, skim milk powder, glycerin, propylene glycol, water, ethanol and the like. The composition may also contain minor amounts of wetting agents, emulsifying agents or pH buffering agents as needed.

The pharmaceutical composition of the invention may be administered in a suitable route. Preferably, the pharmaceutical composition of the invention is administered orally, intravenously, intraarterially, subcutaneously, intraperitoneally, intramuscularly, or transdermally.

For these routes of administration, the composition of the invention may be administered in a suitable dosage form.

The dosage form includes, but not limited to, tablet, capsule, troch, hard candy, powder, spray, cream, ointment, suppository, gel, paste, lotion, aqueous suspension, injectable solution, elixir, and syrup.

Another object of the invention is to provide a kit comprising the vortioxetine analogue of the pinvention or the polymorph or the solvate thereof, or a pharmaceutical composition of the present invention.

### Treatment and use

A further object of the invention is to provide a method of treating depression disorder, particularly major depression disorder, comprising administering an effective amount of a vortioxetine analogue of the present invention or a polymorph or a solvate thereof to an individual in need thereof.

A further object of the invention is to provide a vortioxetine analogue of the present invention or a polymorph or a solvate thereof for use in the treatment of depression disorder, particularly major depression disorder.

A further object of the invention is to provide use of a vortioxetine analogue of the invention or a polymorph or a solvate thereof for the preparation of a medicament for the treatment of depression disorder, particularly major depression disorder.

As used herein, the term "therapeutically effective amount" refers to an amount of a compound that relieves one or more symptoms of the disorder to be treated after administration in a certain extent.

The dosing regimen may be adjusted to provide the best desired response. For example, a single bolus may be administered, a plurality of sub-doses may be administered over time, or the dose may be proportionally reduced or increased as indicated by the exigencies of the therapeutic situation. It is noted that the dose value can be varied with the type and severity of the condition to be alleviated and can include single or multiple doses. It is further understood that the particular dosage regimen will be adjusted over time based on the individual's needs and the administering composition or the professional judgment of the person supervising the composition for any particular individual.

The administered amount of a compound of the invention will depend on the individual to be treated, severity of the disorder or condition, the administration rate, the handling of the compound, and the judgment of the prescribing physician. In general, an effective dose is from about 0.0001 to about 50 mg per kg body weight per day, for example, from about 0.01 to about 10 mg/kg/day (single or divided doses). For a 70 kg person, this may add up to about 0.007 mg/day to about 3500 mg/day, for example, from about 0.7 mg/day to about 700 mg/day. In some cases, a dose level that is not higher than the lower limit of the foregoing range may be sufficient, while in other cases, a larger dose may still be employed without causing any adverse side effects, provided that the larger dose is divided into several smaller doses to be administered throughout the day.

The content or amount of the compound of the invention in the pharmaceutical composition is from about 0.01 mg to about 1000 mg, suitably from 0.1 to 500 mg, preferably from 0.5 to 300 mg, more preferably from 1 to 150 mg, particularly preferably from 1 to 50 mg. For example, 1.5 mg, 2 mg, 4 mg, 10 mg, 25 mg, and etc.

As used herein, the term term "treating", unless otherwise indicated, means reversion, alleviation, inhibition of a disorder or condition to which such a term applies or a progression of one or more symptoms of such disorder or condition, or prevention of such disorder or condition or one or more symptoms of such disorder or condition.

As used herein, the term "Individual" includes human or non-human animals. Exemplary human individuals include a human individual (referred to as a patient) with a disease, such as the diseases described herein, or a normal individual. As used herein, the term "non-human animals" include all vertebrates, such as non-mammals (eg, birds, amphibians, reptiles) and mammals, such as non-human primates, domestic animals and/or domesticated animals (eg, sheep, dogs, cats, cows, pigs, etc.).

### Example

In order to make the purposes and technical solutions of the invention clear, the invention will be further described below with reference to specific examples. It is noted that the following examples are only intended to illustrate the invention and are not to be understood as limiting the scope of the invention. Some non-essential improvements and modifications made by those skilled in the art according to the above description of the invention are within the scope of the invention. Further, specific experimental methods not mentioned in the following examples are carried out in accordance with a conventional experimental method.

As used herein, the abbreviations have the following meanings:

| Abbreviations | Meanings |
|---|---|
| NaNO₂ | Sodium nitrite |
| HPLC | High performance liquid chromatography |
| THF | Tetrahydrofuran |

### Compound preparation example

### A. Preparation of a compound of formula II

### Example 1

### Preparation of 4-trifluoromethyl-2-methylthiophenol

1) 4-Trifluoromethyl-2-methylaniline (10 mmol) was dissolved in ethanol-water (20 ml, 1:1) at 0-5 °C. Concentrated hydrochloric acid (20 mmol) was added dropwise, and an aqueous solution of NaNO₂ (10.05 mmol) in 5 ml of water was slowly added in batches. After the addition was completed, the reactants were stirred at 0-5 °C for 30 min to give a diazonium salt of 4-trifluoromethyl-2-methylaniline for subsequent use.
2) The diazonium salt solution prepared in step 1) was slowly added dropwise to a solution of potassium ethyl xanthate (10.1 mmol) in deionized water (10 ml) at 40-45 °C. The reaction was detected by HPLC. After the reaction was completed, the aqueous layer was extracted with dichloromethane (10 mlx3), and the organic layers were combined and washed with saturated brine (10 ml), dried with anhydrous sodium sulphate, filtered, and evaporated under reduced pressure to remove the solvents. The residue was separated by preparative chromatography to obtain O-ethyl-S-[4-(trifluoromethyl)-2-methylphenyl]dithiocarbonate.
3) O-ethyl-S-[4-(trifluoromethyl)-2-methylphenyl]dithiocarbonate (5 mmol) obtained in step 2) was dissolved in ethanol (20 ml) at room temperature. A solution of potassium hydroxide (1.2 g) in 95% of ethanol (10 ml) was slowly added dropwise to the obtained solution, stirred at room temperature for 30 min. The solvents were evaporated under reduced pressure, the residue was diluted with water (20 ml), and the aqueous layer was washed with diethyl ether (20 mlx4). The pH value of the aqueous layer was adjusted to 2. The obtained aqueous layer was extracted with diethyl ether (20 mlx3). The extraction solution was washed with a saturated sodium chloride solution twice, dried, and evaporated to obtain the title compound.
   ESI-MS [M-H]⁻ 191.17;
   ¹H-NMR (DMSO) δ: 2.36(3H), 3.48(1H), 7.25-7.42(3H).

### B. Preparation of a compound of formula III

### Example 2

### Preparation of 1-tert-butoxycarbonyl-4-[2-(mercapto)phenyl]piperazine (Compound 10)

1) 4-(2-Aminophenyl)-1-tert-butoxycarbonylpiperazine (5 mmol) was dissolved in ethanol-water (20 ml, 1:1) at 0-5 °C. Concentrated hydrochloric acid (10 mmol) was added dropwise, and an aqueous solution of NaNO₂ (5.05 mmol) in 3 ml of water was slowly added in batches. After the addition was completed, the reactants were stirred at 0-5 °C for 30 min to give the diazonium salt of 4-(2-aminophenyl)-1-tert-butoxycarbonylpiperazine for subsequent use.
2) The diazonium salt solution prepared in step 1) was slowly added dropwise to a solution of potassium ethyl xanthate (10.1 mmol) in deionized water (10 ml) at 40-45 °C. The reaction was detected by HPLC. After the reaction was completed, the aqueous layer was extracted with dichloromethane (10 mlx3), and the organic layers were combined and washed with saturated brine (10 ml), dried with anhydrous sodium sulphate, filtered, and evaporated under reduced pressure to remove the solvents. The residue was separated by preparative chromatography to obtain O-ethyl S-[1-tert-butoxycarbonyl-4-phenylpiperazine]dithiocarbonate for subsequent use.
3) The dithiocarbonate (5 mmol) obtained in step 2) was dissolved in ethanol (20 ml) at room temperature. A solution of potassium hydroxide (1.2 g) in 95% of ethanol (10 ml) was slowly added dropwise to the obtained solution, stirred at room temperature for 30 min. The solvent was evaporated under reduced pressure, the residue was diluted with water (20 ml), and the aqueous layer was washed with diethyl ether (20 mlx4). The pH value of the aqueous layer was adjusted to 2. The obtained aqueous layer was extracted with diethyl ether (20 mlx3). The extraction solution was washed with a saturated sodium chloride solution twice, dried, and evaporated to obtain the title compound.
   ESI-MS [M-H]⁻ 276.34;

### Example 3

### Preparation of 1-benzyloxycarbonyl-4-[2-(mercapto)phenyl]piperazine (Compound 11)

Compound 11 was obtained with 1-Benzyloxycarbonyl-4-(2-aminophenyl)piperazine being used as starting material according to the procedure of Example 2.
ESI-MS [M-H]⁻ 310.35;

### Example 4

### Preparation of 1-(9-fluorenylmethoxycarbonyl)-4-[2-(mercapto)phenyl]piperazine (Compound 12)

Compound 12 was obtained with 1-(9-fluorenylmethoxycarbonyl)-4-(2-aminophenyl) piperazine being used as starting material according to the procedure of Example 2.
ESI-MS [M-H]⁻ 398.46;

### C. Preparation of a compound of formula IV

### Example 5

### Preparation of 1-tert-butoxycarbonyl-4-[2-(2-methyl-4-trifluoromethylphenylthio)phenyl] piperazine (Compound 16)

1) 4-(2-Aminophenyl)-1-tert-butyloxycarbonylpiperazine (10 mmol) was dissolved in ethanol-water (20 ml, 1:1) at 0-5 °C. Concentrated hydrochloric acid (20 mmol) was added dropwise, and an aqueous solution (5 ml) of NaNO₂ (10.05 mmol) was slowly added in batches. After the addition was completed, the reactants were stirred at 0-5°C for 30 min to give a diazonium salt of 4-(2-aminophenyl)-1-tert-butoxycarbonylpiperazine for subsequent use.
2) The diazonium salt solution prepared in step 1) was slowly added to the solution of 4-trifluoromethyl-2-methylthiophenol (Example 1, 10 mmol) in ethanol: water (1:1) (10 ml) at 50-60 °C. The reaction was detected by HPLC. After the reaction was completed, the aqueous layer was extracted with dichloromethane (10 mlx3), and the organic layers were combined and washed with saturated brine (10 ml), dried with anhydrous sodium sulphate, filtered, evaporated under reduced pressure to remove the solvents. The residue was separated by preparative chromatography to obtain the title compound.
   ESI-MS [M+H]⁺ 453.52;
   ¹H-NMR (DMSO) δ: 1.37(9H), 2.36(3H), 2.88(4H), 3.10(4H), 6.93(1H), 7.04(1H), 7.18(2H), 7.38(1H), 7.46(1H), 7.65(1H).

### Example 6

### Preparation of 1-benzyloxycarbonyl-4-[2-(2-methyl-4-trifluoromethylphenylthio)phenyl] piperazine (Compound 17)

The title compound was obtained with the compound of Example 1 and 4-(2-aminophenyl)-1-benzyloxycarbonylpiperazine being used as starting materials according to the procedure of Example 5.
ESI-MS [M+H]⁺ 487.53;

### Example 7

### Preparation of 1-(9-fluorenylmethoxycarbonyl)-4-[2-(2-methyl-4-trifluoromethylphenylthio) phenyl]piperazine (Compound 18)

The title compound was obtained with the compound of Example 1 and 4-(2-aminophenyl) -1-(9-fluorenylmethoxycarbonyl)piperazine being used as raw materials according to the procedure of Example 5.
ESI-MS [M+H]⁺ 561.61.

### Example 8

### Preparation of Compound 16 from Compound 10

1) 4-Trifluoromethyl-2-methylaniline (10 mmol) was dissolved in ethanol-water (20 ml, 1:1) at 0-5 °C. Concentrated hydrochloric acid (20 mmol) was added dropwise, and an aqueous solution (5 ml) of NaNO₂ (10.05 mmol) was added slowly in batches. After the addition was completed, the reactants were stirred at 0-5 °C for 30 min to give a diazonium salt of 4-trifluoromethyl-2-methylaniline for subsequent use.
2) The diazonium salt obtained from step 1) was added dropwise to a solution (10 ml) of 1-BOC-4-[2-(sulfhydryl)phenyl] piperazine (Compound 10, 10 mmol) in ethanol-water (1:1) at 50-60 °C. The reaction was detected by HPLC. After the reaction was completed, the mixture was extracted with dichloromethane (10 mlx3), then the organic layers were combined, and washed with saturated brine (10 ml), dried with anhydrous sodium sulphate, filtered, evaporated under reduced pressure to remove the solvents. The residue was separated by preparative chromatography to obtain the title compound.

### Example 9

### Preparation of Compound 17 from Compound 11

The Compound 17 was obtained with 4-trifluoromethyl-2-methylaniline and 1-benzyloxycarbonyl-4-[2-(sulfhydryl)phenyl]piperazine (Compound 11) being used as raw materials according to the procedure of Example 8.

### Example 10

### Preparation of Compound 18 from Compound 12

The Compound 18 was obtained with 4-trifluoromethyl-2-methylaniline and 1-(9-fluorenylmethyloxycarbonyl)-4-[2-(sulfhydryl)phenyl]piperazine (Compound 12) being used as raw materials according to the procedure of Example 8.

### D. Preparation of vortioxetine analogue of the present invention

### Example 11

### Preparation of Compound 1 from Compound 16

1) Compound 16 (5 mmol) obtained in Example 5 was dissolved in dry ether (20 ml) at room temperature, and HCl gas was slowly introduced. The reaction was detected by HPLC. After the reaction was completed, ether was evaporated under reduced pressure to obtain 1-[2-(2-methyl-4-trifluoromethylphenylthio)phenyl]piperazine hydrochloride (Compound 2).
2) Compound 2 (5 mmol) obtained in step 1) was dissolved in water (20 ml). In ice bath, after pH value was adjusted to 10-11 with IN NaOH, the aqueous layer was extracted with dichloromethane (10 mlx3), then the organic layers were combined, and washed with saturated saline (10 ml), dried with anhydrous sodium sulphate, filtered, evaporated under reduced pressure to remove the solvents to obtain 1-[2-(2-methyl-4-trifluoromethylphenylthio)phenyl]piperazine (Compound 1).
   ESI-MS [M+H]⁺ 353.39.
   ¹H-NMR (DMSO) δ: 2.37(3H), 3.30(4H), 3.17(4H), 6.95(1H), 7.08(1H), 7.20(2H), 7.32(1H), 7.48(1H), 7.68(1H); m.p=206-208°C.

### Example 12

### Preparation of Compound 1 from Compound 17

1-Benzyloxycarbonyl-4-[2-(2-methyl-4-trifluoromethylphenylthio)phenyl]piperazine (Compound 17, 10 mmol) was dissolved in THF (50 ml) at room temperature. 5% Pd-C (500 mg) was added, and the air was replaced with hydrogen at room temperature. The reaction was detected by HPLC. After the reaction was completed, the mixture was filtered and the solvent was evaporated under reduced pressure, to obtain Compound 1.

### Example 13

### Preparation of compound 1 from compound 18

In an ice bath, 1-(9-fluorenylmethyloxycarbonyl)-4-[2-(2-methyl-4-trifluoromethyl phenylthio)phenyl]piperazine (Compound 18, 10 mmol) was dissolved in methanol (50 ml). Piperidine (10 mmol) was added, and the ice bath was removed to allow them to react at room temperature. The reaction was detected by HPLC. After the reaction was completed, the solvent was evaporated under reduced pressure. The residue was separated by preparative chromatography to obtain Compound 1.

### Example 14

### Preparation of Compound 3

Under nitrogen, the Compound 1 (10 mmol) obtained from Example 11 was dissolved in anhydrous ethanol (15 ml), and the solution was heated to reflux. A anhydrous ethanol solution (1 ml) containing 40% hydrobromic acid (10 mmol) was added slowly, and allowed to reflux for 15 min. The mixture was allowed to cool naturally to precipitate white solid, filtered, and the filter cake was dried under reduced pressure, to obtain 1-[2-(2-methyl-4-trifluoromethylphenylthio) phenyl]piperazine hydrobromide salt (Compound 3).
m.p.: 170-172°C.

### Example 15

### Preparation of Compound 4

In an ice bath, compound 1 (5 mmol) was dissolved in anhydrous ethanol (20 ml). An ethanol solution (10 ml) containing sulphuric acid (10 mmol) was added slowly, and solid precipitated. The precipitation was filtered to obtain 1-[2-(2-methyl-4-trifluoromethylphenylthio) phenyl]piperazine sulfate (Compound 4).
m.p.: 164-166 °C.

### Example 16

### Preparation of Compound 5

1-[2-(2-methyl-4-trifluoromethylphenylthio)phenyl]piperazine methanesulphonate (Compound 5) was obtained with Compound 1 and methanesulfonic acid being used as raw materials, according to the procedure of Example 15.
m.p.: 152-155 °C.

### Example 17

### Preparation of Compound 6

1-[2-(2-methyl-4-trifluoromethylphenylthio)phenyl]piperazine ethanesulfonate (Compound 6) was obtained with Compound 1 and ethanesulfonic acid being used as raw materials, according to the procedure of Example 15.
m.p.: 149-151 °C.

### Example 18

### Preparation of Compound 7

1-[2-(2-methyl-4-trifluoromethylphenylthio)phenyl]piperazine benzenesulphonate (Compound 7) was obtained with Compound 1 and benzenesulfonic acid being used as raw materials, according to the procedure of Example 15.
m.p.: 145-147 °C.

### Example 19

### Preparation of Compound 8

1-[2-(2-methyl-4-trifluoromethylphenylthio)phenyl]piperazine tosylate (Compound 8) was obtained with Compound 1 and toluenesulfonic acid being used as raw materials, according to the procedure of Example 15.
m.p.: 141-143 °C.

### Example 20

### Preparation of compound 9

1-[2-(2-methyl-4-trifluoromethylphenylthio)phenyl]piperazine citrate (Compound 9) was obtained with Compound 1 and citric acid being used as raw materials, according to the procedure of Example 15.
m.p.: 151-154 °C.

### Biological Examples

### Example 21

### Determination of lipid-water partition coefficient

Vortioxetine hydrobromide (6 mg) and Compound 3 (6 mg) were dissolved respectively in a mixed solution composed of a pH 7.4 of phosphate buffer (3 ml) and n-octyl alcohol (3 ml), and the mixture was allowed to stand for 3 hours at 37 °C. The concentration of each compound in water phase and oil phase was respectively determined, and calculated by logP =-log (C_{oil/} C_{water}). The results are as follows: logPᵥₒᵣₜᵢₒₓₑₜᵢₙₑ=2.1; logP_{compound 3}=2.7.

The lipid-water partition coefficient of the compound of the present invention is larger than that of vortioxetine, indicating that the compound of the present invention has better lipophilic property and can cross the blood brain barrier (BBB) more easily.

### Example 22

### Determination of half maximal inhibition concentration (IC₅₀) of [³H]5-hydroxytryptamine (5-HT) reuptake

SLC6A4 gene from plasmid Pcmv6-XL4-SLC6A4 (Origene, Cat#SC119478) was amplified by PCR (polymerase chain reaction), and a HIS tag was added to the C terminal, and then inserted into pcDNA3.1 (-) vector at 5'EcoR1 and 3'Hind111 restriction enzyme cutting sites to build pcDNA3.1-SLC6A4-His.

CHO cells in logarithmic growth period (Chinese hamster ovary cells, Institute of Cell Biology, Chinese Academy of Sciences, Cat#CCL-61) were collected and counted. The cells were resuspended in F-12 culture media (Gibco, Cat#11330-032) and inoculated on a 96-well plate with 100 µL (20000 cells/well) per well. It was incubated in 5% CO₂ incubator (relative humidity 100%) at 37°C for 24 h.

The above modified plasmids (0.48 µg) were diluted with Opti-MEM culture medium (25 µL, Gibco, Cat#11058021), and blew gently to mix homogenously. FuGENE HD transfection reagent (1.44 µL, Promega, Cat#E2311) was diluted with Opti-MEM culture medium (25 µL, Gibco, Cat#11058021). It was allowed to stand for 5 min at room temperature after being blown gently to mix homogenously. Two solution prepared in the two steps respectively were mixed and blown gently. Then it was allowed to stand for 12 min at room temperature. The transfection complex was added to the 96-well cell plate by 50 µL/well. The cell plate was gently shook to mix evenly and incubated in 5% CO₂ incubator at 37 °C for 48 h.

Compound 1 and vortioxetine were respectively diluted to 1 mM with Opti-MEM culture medium, then diluted 10 times per 4 times gradient, and then added to the transfected cell wells by 20 µL/well (the corresponding concentrations were 100 µM-0.0004 µM, in total of 10 concentration points, the concentration ratio between each concentration point was 4, each concentration included 3 parallel wells). After addition, they were incubated for 30 min, then [³H]5-hydroxytryptamine (25 nM, Perkinelmer, Cat#NET1167250UC) was added. After incubated at 37 °C for 30 min, the cells were lysed with 0.5 M NaOH. 50 µL of lysed cell fluid was taken, and 200 µL of scintillation solution was added thereto, and reading was conducted with MicroBeta2.

The calculation formula is: % inhibition = [100% active wells-sample wells]/100% active wellsx100

The results are shown in the following table:

| Half inhibition | Vortioxetine | Compound 1 |
|---|---|---|
| IC₅₀ | 266.6 nM | 230.6 nM |

The experiment results show that half inhibition concentration of [³H] 5-HT reuptake (IC₅₀) for the compound of the present invention is equivalent to that of vortioxetine, indicating that the compound of the present invention has a 5-HT reuptake inhibitory activity comparable to that of vortioxetine.

### Example 23

### Head-twitch test in mice

KM mice (male, 18-22 g, Experimental Animal Center, Chongqing Medical University) were randomly divided into 6 mice per group. According to the below table, the animals in each experiment group were administrated a solution of corresponding agent, and then 200 mg/kg 5-HTP (5-hydroxytryptophan). The effects of vortioxetine and Compound 3 on the frequency of head-twitch of mice within 0-10 min were determinate (see [J] Brit. J. Pharmacol. (1963), 20, 106-120 for detailed experiment methods). The administration method, dosage, observation time and frequency of head-twitch are shown in the table below:

| Group | Number of animal | Dosage of administration (mg/kg) | Administration method | Number of head-twitch |
|---|---|---|---|---|
| Physiological saline group | 6 | - | Intraperitoneal injection | 2.7±2.1 |
| Compound 3 group | 6 | 20 | intraperitoneal injection | 45.0±29.0 |
| Vortioxetine group | 6 | 20 | Intraperitoneal injection | 56±10 |

1. Compared with the negative control group (physiological saline group), the number of head-twitch increased significantly after intraperitoneal injection of Compound 3, primarily indicating that Compound 3 acted on the central nervous system of 5-HT and inhibited the reuptake of 5-HT neurotransmitter;
2. Compared with vortioxetine, under the same administration method and dosage, after acting on 5-HTP induced head-twitch model, Compound 3 produced stronger tremor intensity and longer tremor period on the model mice than vortioxetine.

The experiment results show that the compound of the present invention has the same inhibitory effect on the reuptake of 5-HT neurotransmitter, and the intensity is stronger than that of vortioxetine.

### Example 24

### Test of brain concentration and plasma concentration

KM mice (male, 18-22 g, Experimental Animal Center, Chongqing Medical University) were randomly divided into groups with 6 mice per group. In accordance with the table below, the animals in each group were respectively injected intravenously with equal moles of vortioxetine (20 mg/kg) and Compound 1 (23.6 mg/kg). Samples of blood and brain tissue of the mouse were taken at 1 and 2 hours after injection. The concentrations of vortioxetine and Compound 1 in blood plasma and brain tissue were respectively measured, and the ratios (concentration in brain tissue/concentration in blood plasma) were calculated. The results are as follows:

| Test material | Dosage (mg/kg) | 1 hour after injection | | | 2 hours after injection | | |
|---|---|---|---|---|---|---|---|
| | | Concentration in brain tissue (µg/g) | Blood drug level (ng/ml) | Concentration ratio in brain tissue and plasma | Concentration in brain tissue (µg/g) | Blood drug level (ng/ml) | Concentration ratio in brain tissue and plasma |
| Vortioxetine | 20 | 15.40±1.40 | 260.95±53.48 | 60.20±8.64 | 7.49±0.56 | 167.24±49.47 | 44.65±13.06 |
| Compound 1 | 23.6 | 40.38±2.96 | 655.40±60.70 | 61.76±2.45 | 35.24±3.75 | 438.39±90.70 | 82.01±12.15 |

The experiment results show that the compound of the present invention crosses the blood-brain barrier more easily than vortioxetine, and its brain exposure is higher than that of vortioxetine.

### Example 25

### Test of blood drug level in rats

SD rats (male, 180-220 g, Experimental Animal Center, Chongqing Medical University) were randomly divided into groups with 4 rats per group. Food was prohibited, but not water, for 8 hours before administration. They were intragastrically administered with the dosage shown in the following table:

| Group | Fasting food time (h) | Dosage (mg/kg) | Drug level (mg/ml) | Dosage of administration (ml/100g) |
|---|---|---|---|---|
| Vortioxetine group | 8 | 20 | 1 | 2 |
| Compound 3 group | 8 | 22.8 | 1.18 | 2 |

Blood samples were collected from the orbital venous plexus at 0, 1, 2, 4, 6, 8 h after administration, wherein at least 0.3 ml was taken at each time point, and heparin was used for anticoagulant. The obtained whole blood (200 µl) was added to acetonitrile (800 µl), vortexed and shaken for 1 min, and then centrifuged for 10 min at 12000 rpm. The supernatant was taken and kept at -20 °C, and the blood drug level of the supernatant was determined by LC/MS/MS. The data for each animal in groups are as follows:

| | | Vortioxetine (20 mg/kg) | | | | Compound 3 (22.8 mg/kg) | | | |
|---|---|---|---|---|---|---|---|---|---|
| Rat No. | | A1 | A2 | A3 | A4 | B1 | B2 | B3 | B4 |
| Blood drug level | 0h | 0 | 0 | 0 | 0 | 0.815 | 2.195 | 1.62 | 0.975 |
| | 1h | 48.59 | 163.07 | 183.96 | 256.56 | 458.925 | 276.675 | 351.38 | 412.775 |
| | 2h | 85.335 | 149.685 | 164.88 | 195.125 | 587.71 | 237.72 | 441.03 | 648.63 |
| | 4h | 34.54 | 90.305 | 94.53 | 164.845 | 726.37 | 181.565 | 334.54 | 297.135 |
| | 6h | 17.095 | 47.74 | 40.91 | 72.6 | 421.06 | 139.79 | 230.075 | 159.715 |
| | 8h | 7.13 | 6.76 | 23.35 | 26.315 | 386.805 | 37.895 | 109.47 | 78.5 |

According to the above results, the blood drug levels of rats orally administrated with Compound 3 were higher than those in the rats orally administrated with equal moles of vortioxetine for all time points, indicating that the compound provided by the present invention is more easily absorbed, and is metabolized more slowly than vortioxetine.

### Example 26

### Forced swimming test in mice

KM mice (male, 38-42 g, Experimental Animal Center, Chongqing Medical University) were randomly divided into groups with 6 mice per group. After fasted for 4 h, the mice were placed in a glass cylinder with a diameter of 18 cm, height of 40 cm, water depth of 15 cm (water temperature 25 °C) to swim for 6 min, then dried with a warm air blower. The animals in each experiment group were administrated a solution of corresponding agent (Oral administration of gastric lavage, 20 mg/kg) according to the table below. After 1 hour of administration, the mice were placed in a glass cylinder with a diameter of 18 cm, height of 40 cm, water depth of 15 cm (water temperature 25 °C) to swim for 6 min. The swimming of the mice was recorded within 6 min by a camera system, and the data were automatically collected and analyzed by Noldus software. The quiescent time during swimming was recorded and counted within the last 4 min (for the experiment method, see THE JOURNAL OF PHARMACOLOGY AND EXPERIMENTAL THERAPEUTICS (2012), Vol. 340, No. 3: 666-675). The results are shown in the following table:

| Group | Number of animal | Administration dosage (mg/kg) | Administration method | Quiescent time (s) |
|---|---|---|---|---|
| Physiological saline group | 6 | - | Oral administration of gastric lavage | 136±13 |
| Vortioxetine group | 6 | 20 | Oral administration of gastric lavage | 136±37 |
| Compound 3 group | 6 | 20 | Oral administration of gastric lavage | 107±31 |

The experiment results show that when the mice were administrated with the same dosage of the compound of the present invention and vortioxetine hydrobromide, Compound 3 provided by the present invention shortened the quiescent time of the mouse, and thus the compound of the present invention has antidepressant effect.

In addition to those described herein, according to the foregoing, various modifications to the present invention will be obvious to those skilled in the art. Such changes are also intended to fall within the scope of the attached claims. References cited in this application (including all patents, patent applications, journal articles, books, and any other public publication) are cited herein in their entirety.

## Claims

1. A compound of Formula I, or a polymorph or a solvate thereof: 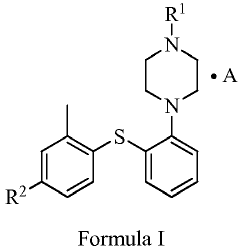 wherein:
R¹ is H;
R² is halogen, or a C₁₋₆ alkyl substituted by one or more halogen atoms, preferably a C₁₋₆ alkyl substituted by one or more fluorine atoms, and more preferably trifluoromethyl; and
A does not exist, or is a pharmaceutically acceptable inorganic or organic acid,
the inorganic acid preferably is selected from hydrochloric acid, hydrobromic acid, hydroiodic acid, sulfuric acid, pyrosulfuric acid, nitric acid, boric acid, phosphoric acid and carbonic acid, and more preferably is hydrochloric acid, hydrobromic acid, or sulfuric acid;
the organic acid preferably is selected from formic acid, acetic acid, propionic acid, acetoacetic acid, trifluoroacetic acid, propionic acid, pyruvic acid, butanoic acids, hexanoic acid, heptanoic acid, hendecanoic acid, lauric acid, stearic acid, palmitic acid, oxalic acid, malonic acid, succinic acid, glutaric acid, adipic acid, maleic acid, fumaric acid, lactic acid, malic acid, citric acid, tartaric acid, benzoic acid, salicylic acid, cinnamic acid, naphthoic acid, pamoic acid, nicotinic acid, orotic acid, methyl sulfuric acid, dodecyl sulfuric acid, methanesulfonic acid, trifluoromethanesulfonic acid, ethanesulfonic acid, ethylene-sulfonic acid, isethionic acid, benzenesulfonic acid, p-toluene sulfonic acid, 1,5-naphthalene disulfonic acid, 2-naphthalene sulfonic acid, camphorsulfonic acid, sulfamic acid, glutamic acid, aspartic acid, gluconic acid and glucuronic acid, and more preferably is citric acid, methanesulfonic acid, ethanesulfonic acid, benzenesulfonic acid, or p-toluene sulfonic acid.

2. The compound or the polymorph or the solvate thereof according to claim 1, wherein the compound is selected from:
| No. | Name |
|---|---|
| 1 | 1-[2-(2-methyl-4-trifluoromethylphenylthio)phenyl]piperazine |
| 2 | 1-[2-(2-methyl-4-trifluoromethylphenylthio)phenyl]piperazine hydrochloride |
| 3 | 1-[2-(2-methyl-4-trifluoromethylphenylthio)phenyl]piperazine hydrobromide |
| 4 | 1-[2-(2-methyl-4-trifluoromethylphenylthio)phenyl]piperazine sulfate |
| 5 | 1-[2-(2-methyl-4-trifluoromethylphenylthio)phenyl]piperazine methanesulphonate |
| 6 | 1-[2-(2-methyl-4-trifluoromethylphenylthio)phenyl]piperazine ethanesulphonate |
| 7 | 1-[2-(2-methyl-4-trifluoromethylphenylthio)phenyl]piperazine benzenesulphonate |
| 8 | 1-[2-(2-methyl-4-trifluoromethylphenylthio)phenyl]piperazine *p*-toluenesulfonate |
| 9 | 1-[2-(2-methyl-4-trifluoromethylphenylthio)phenyl]piperazine citrate |

3. A pharmaceutical composition, comprising the compound or the polymorph or the solvate thereof according to claim 1 or 2, and one or more pharmaceutically acceptable carriers.

4. A kit, comprising the compound or the polymorph or the solvate thereof according to claim 1 or 2, or the pharmaceutical composition according to claim 3.

5. Use of the compound or the polymorph or the solvate thereof according to claim 1 or 2 in the preparation of a medicament for the treatment of depression, especially major depression disorder, wherein the medicament is preferably in a dosage form for oral, intravenous, intra-arterial, subcutaeous, intraperitoneal, intramuscular, or transdermal administration.

6. A method of preparing the compound of claim 1 or 2, comprising the following steps: 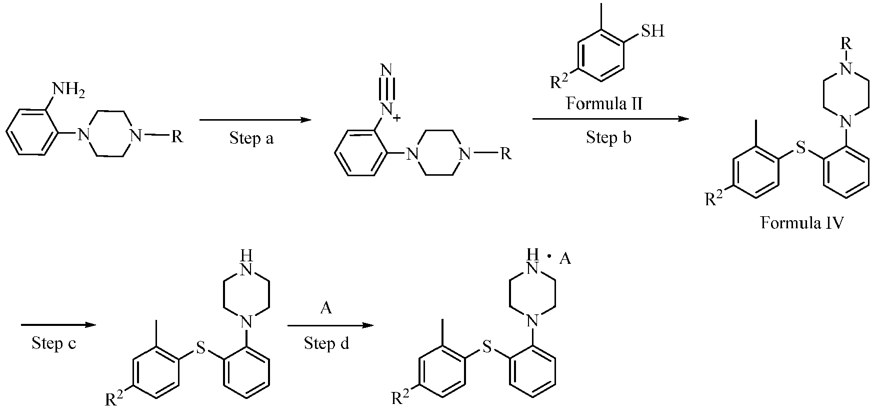 or, 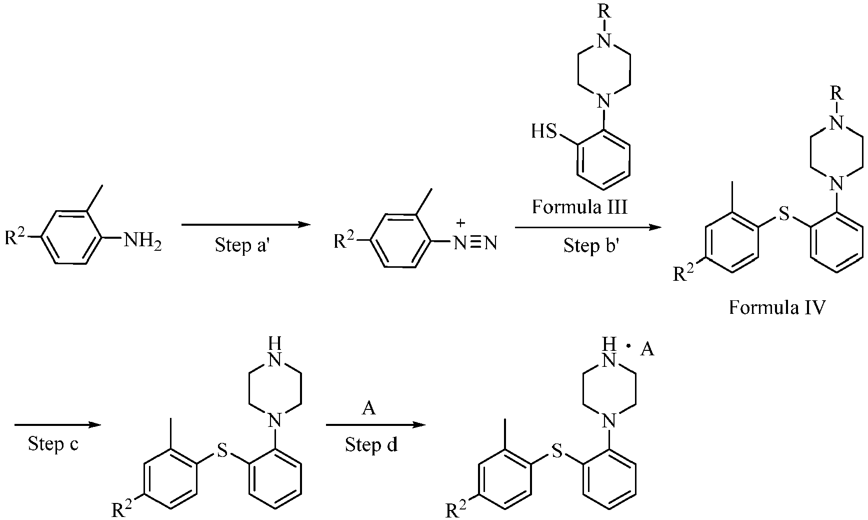 wherein
R is an amino protecting group, preferably is tert-butoxycarbonyl , benzyloxycarbonyl, 9-fluorenylmethoxycarbonyl, allyloxycarbonyl, trimethylsilylethoxycarbonyl, methoxycarbonyl, ethoxycarbonyl, phthaloyl, *p*-toluenesulfonyl, *o*-nitrobenzenesulfonyl, *p*-nitrobenzenesulfonyl, formyl, acetyl, trifluoroacetyl, propionyl, pivaloyl, benzoyl, triphenylmethyl, benzyl, 2,4-dimethoxybenzyl or *p*-methoxybenzyl, more preferably is tert-butoxycarbonyl, benzyloxycarbonyl, 9-fluorenylmethoxycarbonyl, acetyl, propionyl, or benzoyl;
A and R² are defined as in claim 1;
when A does not exist, the reaction of step d is not performed.

7. A compound of Formula IV: 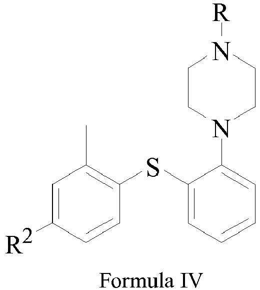 wherein:
R is an amino protecting group, preferably is tert-butoxycarbonyl, benzyloxycarbonyl, 9-fluorenylmethoxycarbonyl, allyloxycarbonyl, trimethylsilylethoxycarbonyl, methoxycarbonyl, ethoxycarbonyl, phthaloyl, *p*-toluenesulfonyl, *o*-nitrobenzenesulfonyl, *p*-nitrobenzenesulfonyl, formyl, acetyl, trifluoroacetyl, propionyl, pivaloyl, benzoyl, triphenylmethyl, benzyl, 2,4-dimethoxybenzyl, or *p*-methoxybenzyl, more preferably is tert-butoxycarbonyl, benzyloxycarbonyl, 9-fluorenylmethoxycarbonyl, acetyl, propionyl, or benzoyl; and
R² is halogen, or a C₁₋₆ alkyl substituted by one or more halogen atoms, preferably is a C₁₋₆ alkyl substituted by one or more fluorine atoms, and more preferably is trifluoromethyl.

8. The compound of claim 7, selected from:
| | |
|---|---|
| 16 | 1-tert-butoxycarbonyl-4-[2-(2-methyl-4-trifluoromethylphenylthio)phenyl]piperazine |
| 17 | 1-benzyloxycarbonyl-4-[2-(2-methyl-4-trifluoromethylphenylthio)phenyl]piperazine |
| 18 | 1-(9-fluorenylmethoxycarbonyl)-4-[2-(2-methyl-4-trifluoromethylphenylthio)phenyl]piperazine |
| 19 | 1-acetyl-4-[2-(2-methyl-4-trifluoromethylphenylthio)phenyl]piperazine |
| 20 | 1-propionyl-4-[2-(2-methyl-4-trifluoromethylphenylthio)phenyl]piperazine |
| 21 | 1-benzoyl-4-[2-(2-methyl-4-trifluoromethylphenylthio)phenyl]piperazine. |

9. A compound of Formula II: 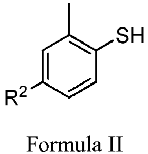 wherein
R² is halogen, or a C₁₋₆ alkyl substituted by one or more halogen atoms, preferably is a C₁₋₆ alkyl substituted by one or more fluorine atoms, and more preferably is trifluoromethyl.

10. A method of preparing the compound of claim 9, comprising the following steps: 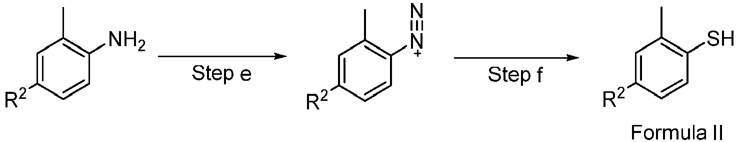 wherein R² is defined as in claim 9.
